# EUROPÄISCHE PATENTANMELDUNG

(11) **EP 0 785 177 A2**
(43) Veröffentlichungstag der Anmeldung: **23.07.1997**
(21) Anmeldenummer: 97100115.1
(22) Anmeldetag: 07.01.1997
(51) Int. Cl.: C07C 5/42, C07B 33/00, C07C 15/46, B01J 8/08

(54) **Verfahren zur Oxidation und Oxidehydrierung von Kohlenwasserstoffen in der Wirbelschicht**

(30) Priorität: 19.01.1996 DE 19601750
(71) Anmelder: BASF AKTIENGESELLSCHAFT, 67056 Ludwigshafen (DE)
(72) Erfinder: Hagemeyer, Alfred, Dr., 48431 Rheine (DE); Schweinze, Jürgen, Dr., 67227 Frankenthal (DE); Watzenberger, Otto, Dr., 67059 Ludwigshafen (DE)

(57) **Zusammenfassung**

Verfahren zur Oxidation und oxidativen Dehydrierung von Kohlenwasserstoffen, insbesondere Ethylbenzol, unter Bildung entsprechender oxidierter bzw. olefinisch ungesättigter Verbindungen, insbesondere Styrol, an einem Sauerstoff übertragenden, mit Sauerstoff regenerierbaren Katalysator, das einen Arbeitstakt, einen zeitlich versetzten Regeneriertakt und mindestens eine zwischengeschaltete Spülphase aufweist, wobei während des Arbeitstakts durch zeitlich versetzte Zugabe einer unterstöchiometrischen Menge an Sauerstoff eine Teilregenerierung vorgenommen wird.

## Beschreibung

Die Erfindung betrifft ein Verfahren zur Herstellung olefinisch ungesättigter Verbindungen wie insbesondere von Styrol durch Oxidation oder oxidative Dehydrierung in der Gasphase mittels Redoxkatalysatoren in der Wirbeischicht.

Styrol (SM) ist ein wichtiges Monomer für technische Kunststoffe und wird in großen Mengen gebraucht. Styrol wird nahezu ausschließlich durch (nichtoxidative) Dehydrierung von Ethylbenzol (EB) bei ca. 600 °C gewonnen. Bei der Dehydrierung handelt es sich um eine Gleichgewichtsreaktion, die in technischem Maßstab so geführt wird, daß ein Umsatz von etwa 60-70 % erreicht und nicht umgesetztes EB danach abgetrennt und zurückgeführt wird. Die Umsetzung verläuft endotherm.

Vollständiger Umsatz ist nur mit Verfahren zu erzielen, die eine Entfernung des Wasserstoffs aus dem Reaktionsgemisch ermöglichen. Zur Überwindung des Gleichgewichts ist daher die - exotherm verlaufende - oxidative Dehydrierung vorgeschlagen worden.

Bei der konventionellen (direkten) oxidativen Dehydrierung wird Sauerstoff mit dem Reaktionsgemisch zugeführt und über einen einzigen, i.d.R. fest angeordneten Katalysator geleitet: Es bildet sich Wasser, aus dem Reaktionsgleichgewicht entfernt wird, so daß nahezu quantitativer Umsatz bei relativ niedriger Temperatur erreicht wird. Da meist mit Sauerstoffüberschuß gearbeitet wird, ist die Ablagerung von Nebenprodukten (Verkokung) auf dem Katalysator nicht allzu kritisch und so kann ein stationärer Prozeß über lange Zeiträume aufrechterhalten und eine hohe Raum-Zeit-Ausbeute (RZA) erreicht werden. Ein Nachteil der oxidativen Dehydrierung sind bisher unvermeidliche Nebenreaktionen, die zur Totaloxidation und damit zum Wertstoffverlust führen.

Zu Vermeidung dieses Nachteils kann man nach einem Vorschlag in der EP-A-0 336 622 mehrere Katalysatorsysteme verwenden, wobei zunächst zwei oder mehr Dehydrierzonen vorgesehen sind und das sauerstoffhaltige Gas an mehreren Stellen eingespeist und anschließend der Produktstrom über einen Oxidationskatalysator geleitet wird. Für die Ethylbenzol-Dehydrierung soll ein konventioneller Eisenoxid-Katalysator mit einem nachgeschalteten Edelmetall-Oxidationskatalysator gekoppelt werden.

Eine andere Möglichkeit, um den direkten Kontakt der Reaktanden mit freiem Sauerstoff zu umgehen, beruht auf der räumlichen oder zeitlichen Trennung der Teilschritte der Reaktion mittels eines als Sauerstoffspeicher bzw. Sauerstoffüberträger wirkenden Redoxkatalysators. Dieses Verfahren wird als instationäre (indirekte) Reaktionsführung bezeichnet und ist für verschiedene chemische Umsetzungen vorgeschlagen worden. Beispiele sind die instationäre Oxidation und Ammonoxidation von Propen, die oxidative Dehydrierung von Alkanen und Alkoholen, die oxidative Dehydrierung von Mono- zu Diolefinen, die oxidative Kopplung von Methan zu höheren Kohlenwasserstoffen, die Dehydrodimerisierung von Toluol zu Stilben, die Dehydrocyclisierung und Dehydroaromatisierung von Paraffinkohlenwasserstoffen, die Butadien-Oxidation und die Butan-Oxidation. Es werden Bi- und V-haltige Redoxkatalysatoren als mögliche Katalysatoren genannt.

Der Redoxkatalysator katalysiert die Kohlenwasserstoff-Oxidationsreaktionen durch Abgabe von Gittersauerstoff unter Bildung von Wasser und wird dabei selbst reduziert. Der reduzierte Katalysator wird anschließend durch molekularen Sauerstoff reoxidiert. Während des Regenerationsschrittes werden auch eventuelle Koksablagerungen auf dem Katalysator entfernt, so daß die Ausgangsaktivität i.a. vollständig wiederhergestellt wird. Der Zyklus wird ständig wiederholt.

Technisch realisiert ist die instationäre Reaktionsführung in Verfahren zur Abgasreinigung, SCR-Entfernung von Stickoxiden und bei der Schwefelsäureherstellung. In technischem Maßstab wird auch bei Cat-Crackern die Crackreaktion und die anschließende Regenerierung des Katalysators zum Abbrennen von Koksablagerungen über zirkulierende Wirbelschichtreaktoren räumlich getrennt. Beim katalytischen sog. Reforming (Isomerisierung von Kohlenwasserstoffen in der Raffinerietechnik) wird ein Wanderbett eingesetzt. Es ist auch vorgeschlagen worden, die räumliche Trennung in einer sog. Riser-Regenerator-Wirbelschicht für die Oxidation von Butan zu Maleinsäureanhydrid einzusetzen und ebenfalls technisch genutzt wird ein instationäres Verfahren zur Dehydrierung von Propan (sog. Catofin-Verfahren). Dabei werden vier getrennte, adiabatisch arbeitende Festbettreaktoren verwendet, die nacheinander die Betriebsmodi Dehydrierung - Spülen - Regenerieren - Spülen durchlaufen.

Nach EP-A-039 737 und EP-A-403 462 kann das Prinzip der instationären Reaktionsführung für die oxidative Dehydrierung von Ethylbenzol zu Styrol verwendet werden, wobei zahlreiche redoxaktive Elemente als Katalysatorkomponenten genannt sind. Bevorzugt ist V/MgO. Die instationäre Dehydrierung von Ethylbenzol ist auch in US 4 067 924 mit Mg-Chromit-Kontakten und in US 3 842 132 mit Bi-Cr-Vanadaten beschrieben.

Nach einem nicht vorveröffentlichten Vorschlag können Bi- und V-haltige Katalysatorsysteme für die oxidative Dehydrierung von Ethylbenzol zu Styrol eingesetzt werden. Bevorzugt ist ein K/Cs/La/Bi/TiO₂-Katalysator.

Bei instationärer Betriebsweise sind Umsatz und Selektivität über den Arbeitszyklus hinweg nicht zeitlich konstant: Zu Beginn befindet sich der Katalysator z.B. im oxidierten Zustand und ist hochaktiv. Die Reaktionsgeschwindigkeit ist entsprechend hoch, was auch eine gewisse Erhöhung des Nebenproduktspiegels (z.B. Vergasung zu Kohlenoxiden) zur Folge hat, verbunden mit rechnerisch geringerer Selektivität. Mit zunehmendem Reduktionsgrad des Katalysators geht die Nebenproduktbildung zurück und die Selektivität verbessert sich stetig bis auf einen jeweils katalysatorspezifischen Endwert. Andererseits wird der Katalysator in dem Maße, wie sein Gittersauerstoff aufgezehrt wird, mehr und mehr deaktiviert, so daß der Umsatz sinkt und der Katalysator schließlich regeneriert werden muß. Es ergibt sich, daß die Styrolausbeute als Produkt aus Selektivität und Umsatz i.a. ein flaches Maximum durchläuft.

Im technischen Maßstab wird man den Katalysator nicht bis zur vollständigen Deaktivierung ausnutzen, sondern die Regenerierung einleiten, solange der Umsatz noch wirtschaftlich akzeptabel ist. Dann kann auch die Regenerierzeit kürzer sein, da der Katalysator nur teilreduziert wurde.

Als Abhilfe gegen die Anfangsvergasung sind partielle Vorreduktion mit H2 oder CO vorgeschlagen worden (EP-A-482 276, JA-A-133 602, JA-A-127 819).

Ein Vorteil der instationär geführten Oxidation und Oxidehydrierung gegenüber der Direktoxidation ist in jedem Falle der Selektivitätsgewinn durch Verminderung der Totalverbrennung, da Reaktanden und Sauerstoff nicht mehr gleichzeitig im Reaktionsgemisch anwesend sind. Ferner ergeben sich Vorteile bei der Aufarbeitung ("integrierter Trennprozeß"). Nachteilig ist andererseits die relativ geringe Raum-Zeit-Ausbeute, da während der Regenerierperiode und in den Spülphasen kein Wertprodukt erzeugt wird: Bei der instationären Oxidehydrierung von Ethylbenzol zu Styrol müßte typischerweise der Zyklus aus jeweils 15 Minuten Dehydrierung und Regenerierung sowie zwei Spülphasen von jeweils 5 Minuten bestehen, sodaß einer produktiven Phase von 15 Minuten Wartzeiten von insgesamt 25 Minuten gegenüberstehen würden. Daher wären große Katalysatormassen und entsprechend große Reaktoren erforderlich.

Aufgabe der Erfindung ist die Steigerung der Raum-Zeit-Ausbeute bei der oxidativen Dehydrierung von Kohlenwasserstoffen unter Bildung entsprechender, olefinisch ungesättigter Verbindungen an einem regenerierbaren, Sauerstoff übertragenden Katalysator. Es wurde gefunden, daß diese und andere Aufgaben gelöst werden in einem Verfahren der oxidativen Dehydrierung von Kohlenwasserstoffen, das einen Arbeitstakt (d.h. eine Zeitphase, während der das Wertprodukt gebildet wird), einen zeitlich versetzten Regeneriertakt und mindestens eine Spülphase aufweist, in dem bereits während des Arbeitstakts durch zeitlich versetzte Zugabe einer unterstöchiometrischen Menge an Sauerstoff eine Teilregenerierung vorgenommen wird.

Man macht sich dabei die überraschende Beobachtung zunutze, daß die Anfälligkeit der oxidativen Dehydrierungsreaktion an einem regenerierbaren, Sauerstoff übertragenden Katalysator gegen Nebenreaktionen bei gleichzeitiger Anwesenheit von Sauerstoff geringer ist, wenn der Katalysator bereits teilreduziert ist, d.h. nicht mehr seine volle Übertragungskapazität besitzt.

Erfindungsgemäß gelingt dies, wenn der Katalysator in einem Wirbelbett angeordnet ist, was vermutlich damit zusammenhängt, daß in diesem Falle eine ständige, vollständige Rückvermischung der Katalysatorpartikel stattfindet, d.h. diese sich stets im gleichen Oxidationszustand befinden, sodaß die Vorteile der bisher bekannten Anordnungen und Verfahrensweisen - hohe Selektivität der instationären und hohe Raum-Zeit-Ausbeute der stationären Reaktionsführung - vereinigt und deren Nachteile weitgehend vermieden werden.

Wirbelschichtreaktoren und ihre Verwendung sind an sich bekannt. Die Grundzüge des Wirbelschichtverfahrens sind z.B. in der Monographie "Fluidization Engineering" von Daizo Kunii und Octave Levenspiel, 2. Aufl. 1991 beschrieben. Einen Überblick über die Theorie der Gas - Feststoff - Fluidisation, die unterschiedlichen Betriebszustände, Aspekte der Auslegung und eine Vielzahl technischer Anwendungsbeispiele findet man bei Perry, Chemical Engineers' Handbook (McGraw-Hill Chem. Engg. Series), 6. Aufl. 1973, Seiten 20 - 75. Einen Gesamtüberblick zum Thema Gas - Feststoff-Fluidisation mit sehr umfangreichen Quellenangaben liefert auch Cheremisinoff, Nicholas, Hydrodynamics of Gas-Solids Fluidization, Houston 1984.

Die Erfindung wird am Beispiel der oxidativen Dehydrierung von Ethylbenzol zu Styrol beschrieben, ist aber auch für andere Umsetzungen mit Vorteil einsetzbar.

Sie eignet sich z.B. zur technischen Durchführung oxidativer Dehydrierungsreaktionen von Aliphaten zu Olefinen; von Mono- zu Diolefinen; von Cycloalkanen zu Aromaten; von Alkoholen zu Aldehyden/Ketonen; von Aliphaten und Olefinen zu Oxygenaten; ferner zu Dehydrocyclisierung und -aromatisierung von Aliphaten und Naphthenen zu Aromaten; zur oxidativen Kopplung von Methan zu C₂-Körpern oder von Toluol zu Stilben; zur Ammonoxidation von Aliphaten und Olefinen; zur Oxidation und Ammonoxidation von Seitenkettenaromaten zu Seitenkettenoxygenaten bzw. Nitrilen.

Wesentliches Kennzeichen der Erfindung ist somit die Kombination aus Katalysator-Anordnung in der Wirbeischicht, Verwendung eines regenerierbarem Redoxkatalysators und Reaktionsführung durch zeitlich verzögerte unterstöchiometrische Sauerstoffzugabe während der Oxidations-/Dehydrierphase (Arbeitstakt). Die erfindungsgemäße Reaktionsführung ist weder der konventionellen stationären Fahrweise (kontinuierliche simultane Zuführung von Edukt und Oxidationsmittel) noch der rein instationären Fahrweise (Zuführung von Edukt in Abwesenheit von gasförmigem Sauerstoff) zuzuordnen, vielmehr kombiniert sie die Vorteile dieser beiden bekannten Techniken und vermeidet deren Nachteile.

Durch verzögerte unterstöchiometrische Sauerstoffzugabe wird der Redoxkatalysator fortwährend im teilreduzierten und daher hochselektiven Zustand gehalten. Dadurch wird deutlich höhere Selektivität als bei der stationären Dehydrierung und ferner eine wesentlich verlängerte Zykluszeit gegenüber der instationären Dehydrierung erreicht. Das Verfahren ist an die Verwendung einer Wirbelschicht gebunden, da nur so ein annähernd gleichmäßiger, räumlich homogener Zustand aller Katalysatorpartikel möglich ist.

Es ist zweckmäßig, den Volumenstrom der Wirbelgase konstant zu halten, indem das Inertgas mit Sauerstoff oder Luft in entsprechender Menge versetzt wird. Praktisch wird somit die unterstöchiometrische Sauerstoffzugabe durch teilweisen Ersatz des - gewöhnlich aus Stickstoff bestehenden - Inertgases z.B. durch Luft bewirkt. Mit der Sauerstoffzugabe kann somit zur Aufrechterhaltung eines konstanten Gesamtgasflusses der Trägergasstrom reduziert werden, was vorteilhafterweise den Inertgasverbrauch senkt.

Da die Erscheinung der Verkokung, d.h. die Bildung von Ablagerungen auf dem Katalysator nicht vollständig vermieden werden kann, wird nach einer von Fall zu Fall zu bestimmenden Zeit der Arbeitstakt abgebrochen und durch eine vollständige Reoxidation abgelöst. Nach Erreichen des reoxidierten Zustandes wird der Katalysator dann zunächst gespült, eine angemessene Zeit unter Ausschluß von Sauerstoff betrieben und schließlich wieder Sauerstoff in unterstöchiometrischer Menge zugegeben.

Eine Nebenerscheinung bei unterstöchiometrischer Sauerstoffzugabe ist das Wärmeverhalten der erfindungsgemäßen Anordnung: In dem Maße, wie der Katalysator reduziert und damit immer selektiver wird, kommt es bei der endothermen Ethylbenzoldehydrierung bisher gewöhnlich zu einer Temperaturabsenkung; die Deaktivierung beschleunigt sich weiter. Da nun aber die Reoxidation des teilreduzierten Metalloxids stark exotherm ist, kommt es bei der erfindungsgemäßen Wiederaufladung des Sauerstoffspeichers zu einer Temperaturerhöhung; diese kann die Mobilität des chemisch eingelagerten Sauerstoffs und damit den Katalysatornutzungsgrad erhöhen, ohne zu Selektivitätseinbußen zu führen. Dadurch kommt es zu einer "in-situ-Temperaturrampe" und die Deaktivierung verlangsamt sich weiter.

Die Verwendung einer Wirbelschicht hat gegenüber dem Festbett auch den Vorteil eines besseren Stoffübergangs, da die Katalysatorpartikel vergleichsweise klein sind, ferner den Vorteil einer geringeren Temperatur-Erhöhung bei der Regenerierung (homogene Temperaturverteilung, keine lokalen hot-spots aufgrund der guten Rückvermischung).

Weitere Vorteile der Wirbelschicht sind der sehr gute Wärmeübergang zwischen dem Wirbelbett und den vorhandenen Wärmeaustauschflächen zur indirekten Wärmeauskopplung z.B. durch Dampferzeugung. Durch das fluide Verhalten des Feststoffes ist ferner eine Ein - oder Ausschleusung des Katalysators im Vergleich zum Festbett leichter möglich. Innerhalb einer Wirbelschicht müssen überdies keine Explosionsgrenzen eingehalten werden, weil die hohe Wärmekapazität des Feststoffes die Ausbreitung einer Expiosionsfront unterdrückt.

Die zeitliche Verzögerung der Sauerstoffinjektion zur Kohlenwasserstoffzuführung kann im speziellen Fall z.B. bis 3600, bevorzugt bis 1800, besonders bevorzugt 30 bis 900 Sekunden betragen und muß von Fall zu Fall experimentell bestimmt werden. Vorzugsweise wird mit der Sauerstoffzufuhr begonnen, wenn die Styrolausbeute ihren Maximalwert gerade überschritten hat und zu sinken beginnt.

Es werden zwischen 1 und 99, bevorzugt 5 bis 95, besonders bevorzugt 10 bis 90 mol-% des für eine stöchiometrische Umsetzung mit dem gleichzeitig vorhandenen oxidierbaren Kohlwasserstoff erforderlichen Sauerstoffs und bezogen auf diesen im Reaktionsraum verfügbar gehalten. Bevorzugt wird Luft als Reoxidationsmittel (Verdünnungseffekt) verwendet, wobei außerdem die für die Einhaltung des Wirbelpunktes erforderliche Gesamt-Gasmenge berücksichtigt werden muß. Außerdem ist zu beachten, daß die Explosionsgrenzen für den betreffenden Kohlenwasserstoff nicht überschritten werden, wenn sauerstoffhaltiges Gas unterhalb des Wirbelbodens zugegeben wird. Diese Einschränkung entfällt, wenn der Sauerstoff separat, d.h. in den Reaktionsraum im Bereich hoher Feststoffkonzentration zugeführt wird.

Die Sauerstoffkonzentration kann mit der Reaktionszeit veränderlich, in Form einer sog "Konzentrationsrampe" gewählt werden, sodaß die fortschreitende Deaktivierung des Sauerstoffspeichers durch zunehmenden Sauerstoffgehalt kompensiert wird (je höher der Reduktionsgrad, desto höher die Luftdosis), bis zur annnähernd stöchiometrischen Sauerstoffkonzentration. Je niedriger das Verhältnis von Kohlenwasserstoff zu Sauerstoff, desto geringer die Verkokung und damit umso länger die Standzeit.

Die Anwendung des erfindungsgemäßen Verfahrens setzt voraus, daß keine nennenswerte Oxidation des Kohlenwasserstoffs in der homogenen Gasphase erfolgt, daß die Reoxidationsgeschwindigkeit des reduzierten Katalysators ausreichend hoch ist und der Sauerstoff ausreichend schnell ins Gitter eingebaut wird (kein unselektiver chemisorbierter Sauerstoff an der Katalysatoroberfläche).

Der Katalysator soll über eine hohe Abriebfestigkeit verfügen und eine Rückhaltung oder Rückführung des mit dem Wirbelgasstrom mitgerissenen Feinanteils des Katalysators ist erforderlich.

Geeignete Katalysatoren sind bereits verfügbar. Sie können trägerfrei (d.h. als Vollkatalysator), mit einem Bindemittel oder auf einen Träger, ausgewählt aus der Gruppe der Tone, PILC, Zeolithe, Aluminiumphosphate, Siliciumcarbid und -nitrid, Bornitrid, sowie der Metalloxide, ausgewählt aus der Gruppe Al, Ba, Ca, Mg, Th, Ti, Si, Zn, Cr oder Zr, aufgebracht sein. Der wirksame Bestandteil (Aktivkomponente) besteht aus wenigstens einem Oxid, ausgewählt aus der Gruppe der Oxide der mehrere Oxidationsstufen annehmenden Elemente Ag, As, Bi, Ce, Co, Cr, Cu, Fe, In, Mn, Mo, Nb, Ni, Sb, Sn, Pb, U, V und W. Bevorzugt sind Bi- und V-haltige Redoxkatalysatoren. Ebenso können Mischungen und Umsetzungsprodukte der genannten Elemente verwendet werden. Die Katalysatoren können weiterhin Promotoren, insbesondere (Erd-)Alkali und/oder Seltene Erden enthalten.

Besonders bevorzugt für die Dehydrierung von Ethylbenzol ist ein auf einem TiO₂-Träger aufgebrachter Katalysator, der im wesentlichen Bismuth, Kalium und Lanthan enthält.

Die Redoxkatalysatoren werden nach bekannten Methoden hergestellt wie z.B. durch Trockenmischen, Aufschlämmen, Imprägnieren, Fällung, Cofällung, Sprühtrocknung und anschließende Calcinierung, d.h. Erhitzung auf eine Temperatur von 300 bis 1000°C, die einheitlich oder stufenweise vorgenommen werden kann. Die benötigten Rohstoffe können z.B. als Oxide, Hydroxide, Carbonate, Acetate, Nitrate oder generell Salze der jeweiligen Elemente mit anorganischen oder organischen Anionen vorliegen. Auch Übergangsmetallkomplexe können verwendet werden. Die Calcinierung erfolgt bei Temperaturen, bei denen die jeweiligen Rohstoffe den Katalysator bilden. Die Aktivmasse des Katalysators kann auf einen Träger aufgebracht sein oder mit einem Bindemittel abgeinischt sein. Dazu zählen auch Oberflächenausrüstungen wie z.B. poröse Silikatschichten zur Verbesserung der Abriebfestigkeit des Wirbelgutes.

Wenn Selten-Erdmetalle zur Unterstützung der Wirkung verwendet werden sollen, sollte speziell bei Verwendung von Lanthan nicht vom Oxid, La₂O₃ ausgegangen werden, da dann die Wirkung nur gering ist. Stattdessen sollten La-Oxid-Carbonat, La(OH)₃, La₂(CO₃)₃ oder organische Lanthan-Verbindungen, wie La-Acetat, La-Formiat oder La-Oxalat eingesetzt werden, die bei der Calcinierung zu einer feinverteilten und oberflächenreichen aktiven La-Phase führen. Eine bevorzugte Calciniertemperatur zur Zersetzung von La(Ac)₃ zur aktiven La-Phase ist beispielsweise 550 bis 700°C.

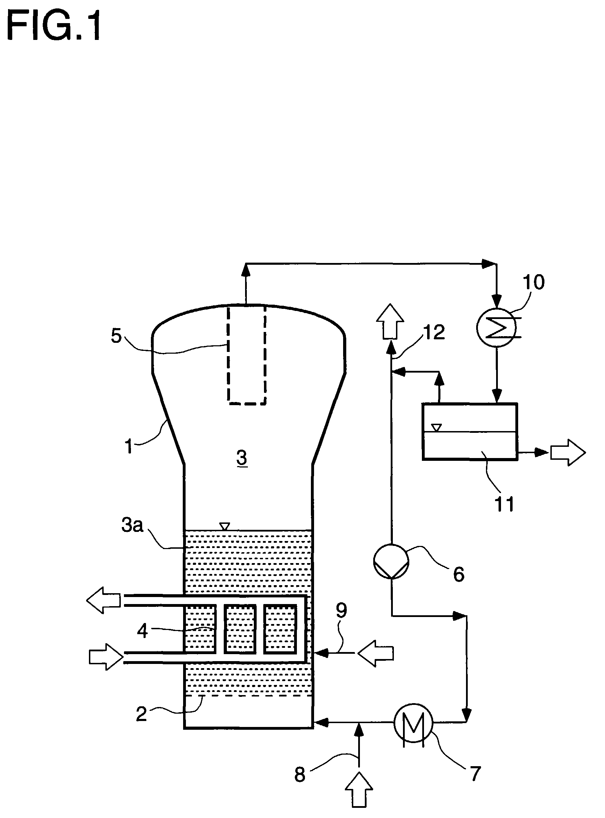

Als Wirbelschichtreaktor zur Durchführung des erfindungsgemäßen Verfahrens eignet sich z.B. der in Abb.1 dargestellte Reaktor (1), der von zylindrischer, im oberen Bereich erweiterter Gestalt ist und zweckmäßig einen Gasverteilerboden (2) aufweist, über dem die Katalysatorschüttung (3a) fluidisiert wird.

Im Reaktionsraum (3) kann ein Wärmetauscher, z.B. in Form von Rohrschlangen oder einem Rohrbündel (4) angeordnet sein, der bei allen Betriebszuständen zur Einstellung der jeweils verlangten Temperatur dient. Die Anordnung des Wärmetauschers kann vertikal, horizontal, spiralförmig oder eine Kombination aus den verschiedenen Orientierungen sein. Als Wärmeträgermedium können Wasser, Dampf, Wärmeträgeröle oder Salzschmelzen Verwendung finden.

Im Reaktor kann eine Feststoffrückhaltung (5) z.B. in Form von Filterkerzen oder Zyklonen eingebaut sein. Die Feststoffabscheidung kann aber ebenso außerhalb des Reaktors erfolgen, mit oder ohne Rückführung des abgeschiedenen Feststoffes in den Reaktionsraum.

Das den Reaktor gasförmig verlassende Produkt wird über eine geeignete Abscheidung (10 und 11 ) z.B. in Form einer Totalkondensation vom Trägergasstrom getrennt. Das Trägergas kann über einen Verdichter (6) und Wärmetauscher (7) wieder in den Reaktor zurückgeführt werden (sog. Kreisgasbetrieb) oder, nach Bedarf, über eine Gasschleuse (12) aus dem System entfernt werden. Der Rohstoff (das Edukt) kann wahlweise über die Einspeisestelle (8) unterhalb des Anströmbodens, gegebenenfalls über eine Vorverdampfung zugeführt werden oder aber (9) direkt flüssig, dampfförmig oder teilkondensiert in den Reaktionsraum zugegeben werden.

Auch das Reoxidationsmittel kann unterhalb oder oberhalb des Anströmbodens (8) oder (9) dem Trägergas oder aufgeteilt zwischen (8) und (9) beigemischt in den Reaktor eingebracht werden.

Die Gasgeschwindigkeit, bezogen auf Leerrohr, liegt z.B. zwischen 0,02 und 1,5 m/s. Bevorzugt ist eine Gasgeschwindigkeit im Bereich zwischen 0,05 und 0,45 m/s. Die Ruheschüttungshöhe, ausgedrückt durch das Verhältnis H/D zwischen Höhe H und Reaktordurchmesser D kann zwischen 0,1 und 10 liegen. Bevorzugt ist eine Höhe, die einem H/D Verhältnis von 0,5 bis 5 entspricht.

Bevorzugt ist bei Flüssigkeiten die Zufuhr direkt in den Reaktionsraum mit Hilfe von Einstoff- oder Zweistoffdüsen.

Der Druckverlust im Gasverteilerboden P_{B} wird zweckmäßig an den Druckverlust in der Wirbelschicht P_{W} angepaßt. Bevorzugt ist ein Verhältnis P_{B}/P_{W} zwischen 0,05 und 0,5, bevorzugt 0,1 bis 0,3.

Die oxidative Dehydrierung von Ethylbenzol wird im Temperaturbereich zwischen 200 und 800°C, bevorzugt 350 bis 600°C, im Druckbereich von 100 mbar bis 10 bar, bevorzugt 500 mbar bis 2 bar, mit einer (für Ethylbenzol) Raumgeschwindigkeit (LHSV) von 0,01 bis 20 h⁻¹, bevorzugt 0,1 bis 5 h⁻¹ durchgeführt. Je nach Rohstoff kann dieser der Wirbelschicht allein oder mit einem entsprechenden Verdünnungsmittel zugeführt werden; im einfachsten Falle ist dies das Trägergas selbst wie beispielsweise CO₂, N₂, ein Edelgas oder Wasserdampf.

Die Regenerierung der teildeaktivierten Katalysatoren wird bei Temperaturen im Bereich 100 bis 800°C, vorzugsweise 250 bis 600°C mit einem freien Oxidationsmittel, vorzugsweise mit N₂O oder einem sauerstoffhaltigen Gas einschließlich reinem Sauerstoff durchgeführt. Auch hier können Verdünnungsmittel im Reaktorzustrom enthalten sein. Geeignet sind außerdem z.B. Luft oder Magerluft. Die Regenerierung kann bei vermindertem Druck, atmosphärischem oder überatmosphärischem Druck betrieben werden. Bevorzugt sind auch hierbei Drucke im Bereich 500 mbar bis 10 bar.

### Beispiele:

### Herstellung des Katalysators

Nach den Angaben der DE-A-44 23 975 wird ein Trägerkatalysator vom Typ K₂O/La₂O₃/Bi₂O₃/TiO₂ verwendet, der folgendermaßen erhalten wird:

K-Carbonat, La-Acetat, basisches Bi-Carbonat ([BiO]₂CO₃), und ein handelsüblicher TiO₂-Träger (DT-51; Rhone-Poulenc) werden trocken gemischt und zunächst trocken, dann unter Zusatz von Wasser und einem Porenbildner für 2,5 h im Kneter verdichtet. Die Knetmasse wird im Extruder zu 3mm-Strängen verformt. Es wird für 16 h bei 120°C getrocknet und für 5 h bei 600°C calciniert. Es werden hellgelbe Stränge der (Gewichts-)Zusammensetzung 12.5% K₂O - 10% La₂O₃ - 25% Bi₂O₃ - 52.5% TiO₂ erhalten.

Die BET-Oberfläche der Stränge beträgt 20,6 m²/g, die Schnitthärte 11 N je Strang.

Die Stränge werden gesplittet und eine Partikelgrößenfraktion von 0,064 mm bis 0,4 mm ausgesiebt.

### Reaktorversuche:

Die katalytische oxidative Dehydrierung von EB zu SM wird in einem Laborwirbelschichtreaktor durchgeführt.

### Versuchsaufbau

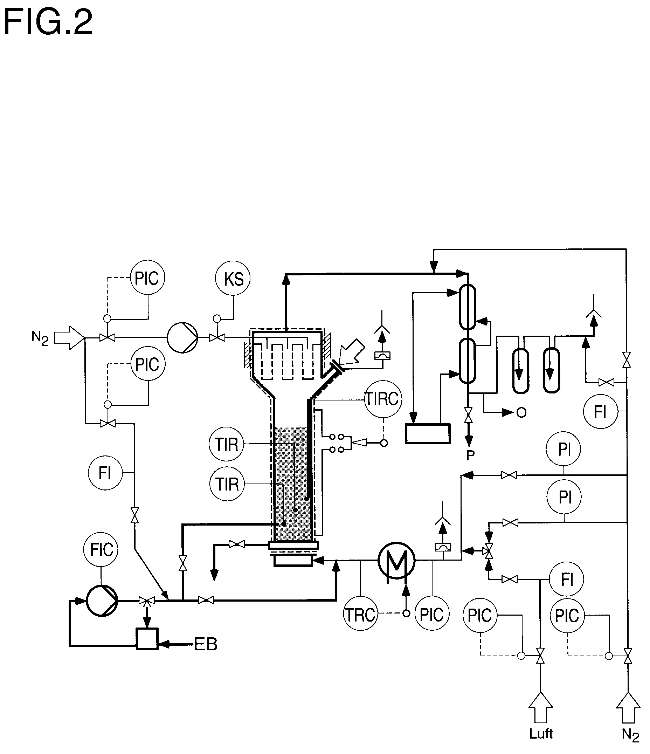

Der Versuchsaufbau ist in Abb.2 dargestellt. Der Wirbelschichtreaktor hat einen Innendurchmesser von 60 mm und eine Höhe von 700 mm im zylindrischen Teil. Zur Reduzierung des Feststoffaustrages und zur Aufnahme keramischer Filterkerzen für die Feststoffabscheidung ist der Reaktor oben konisch erweitert (Freeboard). Der zylindrische Teil des Reaktors ist aus Quarzglas gefertigt und wird durch außen aufgelegte Heizwicklungen elektrisch beheizt. Als Anströmboden (Gasverteiler) dient eine Metallfritte.

Das Fluidisiergas (N₂, Luft oder Mischung) wird mit Rotametern mengenmäßig erfaßt und über einen elektrisch betriebenen, temperaturgeregelten Gaserhitzer unterhalb des Anströmbodens in den Reaktor geleitet. Ethylbenzol wird mittels einer HPLC-Pumpe in den Gasstrom zwischen Gaserhitzer-Austritt und Wirbelbett-Eintritt über eine Kapillare zudosiert. Die den Reaktor gasförmig verlassenden Reaktionsprodukte werden mit zwei in Reihe geschalteten Intensivkühlern kondensiert. Am unteren Ende des zweiten Kühlers befindet sich eine Probenahmestelle P und eine Einrichtung O zur Messung des Sauerstoffgehalts. Zur Abscheidung kondensierbarer Restgase sind zwei mit Trockeneis betriebene Kühlfallen nachgeschaltet.

Die Befüllung des Reaktors erfolgt über einen seitlichen Stutzen im Bereich des Freeboards. Über einen radial angeordneten Stutzen, unmittelbar oberhalb des Anströmbodens kann Katalysator abgezogen werden.

Im Reaktor sind 3 Thermoelemente zur Erfassung der Reaktionstemperatur über die Höhe versetzt angeordnet. Das oberste ist gleichzeitig auf den Regelkreis für die Mantelbeheizung aufgeschaltet. Die Filterreinigung durch Rückblasen erfolgt über eine Zeitsteuerung.

### Versuchsdurchführung, Versuchsbedingungen

Der Reaktor wird mit Katalysator befüllt (1625 g bzw. 1542 ml) und mit vorerhitztem Stickstoff sowie durch Mantelheizung auf die Starttemperatur von 490 °C bis 550°C für die Reoxidation (Regenerierung) aufgeheizt. Die eingestellte Gasmenge liegt bei 430 Nl/h, entsprechend einer Leerrohrgasgeschwindigkeit von 12,6 cm/s. Nach Erreichen stationärer Bedingungen wird dem Fluidisiergas Luftsauerstoff zugemischt. Dabei steigt die Temperatur im Reaktor an und erhöht sich nach ca. 20 Minuten um 30 bis 40 K, abhängig von der Starttemperatur, dem Reduktionsgrad aus dem vorangegangenen Versuch sowie der zugeführten Sauerstoffmenge. Die Sauerstoffzufuhr wird solange aufrecht erhalten, bis das Temperaturmaximurn überschritten und die Temperatur wieder stationär geworden ist. Dadurch wird das Ende des Oxidationsvorganges angezeigt.

An die Oxidationsphase schließt sich eine kurze Phase der Inertisierung mit N₂ an, wobei bei Bedarf bis auf die neue Starttemperatur für die Reaktion hochgeheizt werden kann. Nach Erreichen des stationären Zustandes wird mit der Zudosierung des Ethylbenzols begonnen (Beginn der Dehydrierphase). Die Zulaufmengen liegt bei 7,71 ml EB/min.

2 Minuten nach Beginn der Zufuhr von Ethylbenzol wird zusätzlich 63 Nl/h Luft zugemischt und die entsprechende Stickstoffmenge entsprechend verringert, sodaß die Gasgeschwindigkeit und damit die Verweilzeit konstant bleibt.

Die Luftzufuhr bleibt 11 Minuten geöffnet. Daran anschließend werden die Versuche weitere 2 Minuten nur mit N₂ als Wirbelgas betrieben. Insgesamt wird während dieser Versuche 15 Minuten lang Ethylbenzol dosiert und die Probenahme entsprechend fortgesetzt. Der Zyklus wird geschlossen durch erneute Reoxidation wie oben beschrieben.

Der Vergleichsversuch (rein instationärer Betrieb) unterscheidet sich im wesentlichen dadurch, daß während der gesamten Zulaufzeit von Ethylbenzol keine Luft zugegeben wird und die Zulaufzeit für Ethylbenzol insgesamt nur 5 Minuten beträgt.

Der verflüssigte Austrag wird in Ampullen gesammelt und auf Ethylbenzol, Styrol und Nebenprodukte (Benzol und Toluol) untersucht. Die jeweiligen Gehalte werden in den folgenden Tabellen in Gew.-% angegeben.

Durch Reoxidation der deaktivierten reduzierten Katalysatoren ließ sich die katalytische Aktivität in vollem Umfang wiederherstellen: Über die mehr als 40 vermessenen Zyklen wurde kein mit der Betriebsdauer fortschreitender Aktivitätsverlust festgestellt.

### Beispiel 1 (erfindungsgemäß)

| Probenahme Umsatz [Minuten] [%] | Styrolausbeute [%] | |
|---|---|---|
| 3,5 | 98,85 | 78,46 |
| 4,5 | 98,71 | 79,49 |
| 5,5 | 98,71 | 80,98 |
| 6,5 | 98,47 | 82,02 |
| 7,5 | 98,33 | 82,97 |
| 8,5 | 98,20 | 83,52 |
| 9,5 | 98,06 | 83,67 |
| 10,5 | 97,92 | 83,88 |
| 11,5 | 97,78 | 84,14 |
| 12,5 | 97,69 | 84,04 |
| 13,5 | 97,66 | 84,04 |
| 14,5 | 97,67 | 83,74 |
| 15,5 | 97,70 | 83,74 |
| 16,5 | 97,77 | 83,67 |
| 17,5 | 97,74 | 83,64 |
| 20,5 | 97,88 | 82,33 |

### Vergleichsversuch (rein instationär)

| Probenahme Umsatz [Minuten] [%] | Styrolausbeute [%] | |
|---|---|---|
| 3 | 98,22 | 81,42 |
| 3,5 | 98,02 | 81,76 |
| 4 | 97,88 | 82,46 |
| 4,5 | 97,81 | 83,40 |
| 5 | 97,78 | 84,23 |
| 5,5 | 97,75 | 84,79 |
| 6 | 97,76 | 85,35 |
| 6,5 | 97,74 | 85,66 |
| 7 | 97,73 | 86,52 |
| 7,5 | 97,73 | 87,70 |
| 8 | 97,74 | 88,66 |
| 8,5 | 97,79 | 89,29 |
| 9 | 97,82 | 91,49 |

### Fazit:

Mit verzögerter unterstöchiometrischer Sauerstoffzugabe (Katalysator im teilreduzierten Zustand) konnte die Dehydrierphase gegenüber der rein instationären Fahrweise um den Faktor 2 bis 3 verlängert werden, wobei eine maximale Styrol-Ausbeute von ca. 84% bei knapp 98% Umsatz erzielt wurde. Die Selektivität (nicht optimiert) ist etwas geringer als bei instationärer Betriebsweise.

## Patentansprüche

1. Verfahren zur Oxidation und oxidativen Dehydrierung von Kohlenwasserstoffen unter Bildung entsprechender oxidierter bzw. olefinisch ungesättigter Verbindungen an einem Sauerstoff übertragenden, mit Sauerstoff regenerierbaren Katalysator, das einen Arbeitstakt, einen zeitlich versetzten Regeneriertakt und mindestens eine zwischengeschaltete Spülphase aufweist, dadurch gekennzeichnet, daß während des Arbeitstakts durch zeitlich versetzte Zugabe einer unterstöchiometrischen Menge an Sauerstoff eine Teilregenerierung vorgenommen wird.

2. Verfahren nach Anspruch 1, dadurch gekennzeichnet, daß die Reaktion derart geführt wird, daß zu Beginn des Arbeitstakts dem Wirbelschichtreaktor zuerst der oxidierbare Kohlenwasserstoff in Abwesenheit von freiem Oxidationsmittel zugeführt wird und mit dem Redoxkatalysator in Kontakt tritt, wobei dieser teilreduziert und der Kohlenwasserstoff oxidiert wird und mit einer zeitlichen Verzögerung von bis zu 3600 s ein freien Sauerstoff enthaltender Gasstrom zusätzlich zugeführt wird.

3. Verfahren nach Anspruch 1, dadurch gekennzeichnet, daß der Volumenstrom der Wirbelgase unabhängig von der An- oder Abwesenheit von Sauerstoff konstant gehalten wird.

4. Verfahren nach Anspruch 1, dadurch gekennzeichnet, daß zu jedem Zeitpunkt der Umsetzung eine bezogen auf den jeweils im Reaktionsraum vorhandenen oxidierbaren Kohlenwasserstoff unterstöchiometrische Menge von 10-90 Mol-% Sauerstoff zugeführt wird.

5. Verfahren nach Anspruch 1, dadurch gekennzeichnet, daß die Wirbelschicht mindestens einen Metalloxid-Redoxkatalysator als Sauerstoffüberträger enthält, ausgewählt aus den Oxiden von Bi, V, Ce, Fe, In, Ag, Cu, Co, Mn, Pb, Sn, Mo, Sb, As, Nb, U, W oder deren Mischungen, der gegebenenfalls mit einem anorganischen Bindemittel versehen oder auf einen Träger aufgebracht ist, ausgewählt aus der Gruppe der Tone, Zeolithe, SiC, Si₃N₄, AlPO's, PILC's oder Metalloxide ausgewählt aus der Gruppe der Oxide von Ti, Zr, Zn, Th, Mg, Ca, Ba, Si, Al, Cr oder deren Mischungen.

6. Verfahren nach Anspruch 1, dadurch gekennzeichnet, daß eine alkylaromatische Verbindung oder ein Paraffinkohlenwasserstoff zu dem entsprechenden Alkenylaromaten bzw. Olefin dehydriert wird.

7. Verfahren nach Anspruch 6, dadurch gekennzeichnet, daß Ethylbenzol zu Styrol dehydriert wird.

8. Verfahren nach Anspruch 1, dadurch gekennzeichnet, daß als Oxidationsmittel N₂O anstelle von Sauerstoff verwendet wird.

9. Verfahren nach Anspruch 1, dadurch gekennzeichnet, daß eine zyklische und periodische Reaktionsführung eingehalten wird derart, daß sich an den Arbeitstakt, gegebenenfalls nach einer Spülphase, eine Regenerierphase anschließt, in der der reduzierte Katalysator mit einem Oxidationsmittel reoxidiert wird und gleichzeitig Koksablagerungen auf dem Katalysator abgebrannt werden.

10. Verfahren nach Anspruch 1, dadurch gekennzeichnet, daß die oxidative Dehydrierung von Ethylbenzol im Temperaturbereich zwischen 200 und 800°C bei einem Druck von 100 mbar bis 10 bar mit einer Flüssig-Raumgeschwindigkeit (liquid hourly space velocity; LHSV) von 0,01 bis 20 h⁻¹ durchgeführt wird.

11. Wirbelschichtreaktor zur Durchführung des Verfahrens nach Anspruch 1, dadurch gekennzeichnet, daß ein Reaktor (1) in Form einer zylindrischen, im oberen Bereich erweiterten Wirbelkammer (3) vorhanden ist, der über einen Gasverteilerboden (2) und eine Feststoffrückhaltung (5) verfügt.

12. Wirbelschichtreaktor nach Anspruch 11, dadurch gekennzeichnet, daß der Reaktor (1) über ein innenliegendes Wärmetauscherbündel (4) verfügt.

13. Verwendung des Verfahrens nach Anspruch 1 zur oxidativen Dehydrierung
von Aliphaten zu Olefinen;
von Mono- zu Diolefinen;
von Cycloalkanen zu Aromaten;
von Alkoholen zu Aldehyden/Ketonen;
von Aliphaten und Olefinen zu Oxygenaten.

14. Verwendung des Verfahrens nach Anspruch 1 zur Dehydrocyclisierung und -aromatisierung von Aliphaten und Naphthenen zu Aromaten.

15. Verwendung des Verfahrens nach Anspruch 1 zur oxidativen Kopplung von Methan zu C2-Körpern; von Toluol zu Stilben.

16. Verwendung des Verfahrens nach Anspruch 1 zur Ammonoxidation von Aliphaten und Olefinen.

17. Verwendung des Verfahrens nach Anspruch 1 zur Oxidation und Ammonoxidation von Seitenkettenaromaten zu Seitenkettenoxygenaten bzw. Nitrilen.
